# EUROPEAN PATENT SPECIFICATION

(11) **EP 3 810 127 B1**
(45) Date of publication and mention of the grant of the patent: **04.03.2026**
(21) Application number: 19739764.9
(22) Date of filing: 14.06.2019
(51) Int. Cl.: A61K 31/365, A61P 33/14

(54) **USE OF MILBEMYCIN OXIME AGAINST SEA LICE ON FISH**
VERWENDUNG VON MILBEMYCINOXIM GEGEN SEELÄUSE BEI FISCH
UTILISATION DE L'OXIME DE MILBÉMYCINE CONTRE LES POUX DE POISSON

(30) Priority: 19.06.2018 US 201862686707 P
(43) Date of publication of application: 28.04.2021
(73) Proprietor: Pharmaq AS, 7863 Overhalla (NO)
(72) Inventor: ANDERSEN, Rachmilla Souhoka, 7863 Overhalla (NO); FEIJTER KARLSEN, Marius Andre De, 7863 Overhalla (NO); WIIK-NIELSEN, Christer Ross, 7863 Overhalla (NO)
(74) Representative: Zoetis
(86) International application number: PCT/US2019/037143
(87) International publication number: WO 2019/245891

(56) References cited:
- WO-A1-92/08352
- WO-A2-99/63824
- TANG K.N. ET AL.: "Safety and Efficacy of Milbemycin Oxime and Lufenuron (Sentinel) for Treating Argulus sp. in Two Stingray Species: the Magdalena River Stingray (Potamotrygon magdalenae) and the Smooth Back River Stingray (Potamotrygon orbignyl)", IAAAM 4TH ANNUAL MEETING & CONFERENCE PROCEEDINGS ONLINE, May 2018 (2018-05-01), IAAAM 4th Annual Meeting & Conference 19 - 23 May 2018, Long Beach, pages 1 - 2, XP002793988
- KILLINO TODD J ET AL: "Safety of milbemycin as an oral or bath treatment for the tropical freshwater angelfish Pterophyllum scalare", JOURNAL OF ZOO AND WILDLIFE MEDICINE, vol. 28, no. 1, 1997, pages 94 - 96, XP009515789, ISSN: 1042-7260
- MCNAIR CAROL M: "Ectoparasites of medical and veterinary importance: drug resistance and the need for alternative control methods", JOURNAL OF PHARMACY AND PHARMACOLOGY, vol. 67, no. 3, Sp. Iss. SI, March 2015 (2015-03-01), pages 351 - 363, XP002793990

## Description

The present disclosure describes the use of milbemycin oxime for use in the control of sea lice infestation on fish. More specifically, the disclosure describes a method for controlling sea lice by immersing the fish in water comprising a veterinary effective amount of milbemycin oxime. The invention is defined in the appended claims.

### Background

Sea lice are parasitic crustaceans/copepods within the order *Siphonostomatoida,* family *Caligidae* that feed on the mucus, epidermal tissue, and blood of host marine fish. Johnson et al, Parasitol Res (2002) 88: 789-796. Sea lice are prevalent parasites, particularly on salmonids, and, when present in high numbers, can cause serious infestation, stress, and ultimately host death due to secondary infections. At fish farms, where highly concentrated fish populations are present, sea lice can have a devastating effect on the stock.

According to Kontali Analyse, the total salmonid marine production in 2017 was 2.1 million tons, worth 13.8 billion EUR (€). Available data indicates sea lice cost from €0.1 to €0.2 kg⁻¹ of fish. Mark J Costello, The global economic cost of sea lice to the salmonid farming industry, Journal of Fish Diseases, v. 32(1), pgs 115-118 (2009). However, without treatment measures, sea lice would cost the industry at least four times more and probably increase to levels such as to cause significant direct and indirect mortality to stock. Mustafa et al, Canadian Veterinary Journal 42, 54-56 (2001). Existing regional estimates for the cost of sea lice ranged from 4% of production value for Atlantic Canada to 7-10% in Scotland. Rae et al, Pest Management Science 58, 515-520 (2002)). Notably, Costello et al., *supra,* indicates a cost of 6% of the value of fish production for the countries affected by sea lice.

To date, available treatment regimens against sea lice infestations have been limited. Bath treatments with ALPHA MAX^{®} (deltamethrin), Salmosan^{®} (azamethiphos), and hydrogen peroxide have been used frequently to combat infestations of sea lice. Over time, reduced sensitivity against these compounds has been observed. SLICE^{®} (emamectin benzoate) is used as an in-feed treatment (US Patent No. US 6,486,128). Other ectoparasiticidal compounds have been assessed, such as those described in U.S. Patents 8,128,943 and 6,538,031, but no successful treatment agents have emerged. Studies have also been conducted on vaccine compositions targeting antigens present in sea lice, but no effective products using the vaccine approach have yet to emerge. A milbemycin derivative (23(S)-t-butyloxyimino-25(S)-t-butyl milbemycin X (VS55759) described in WO1992/08352 was shown to be efficacious against sea lice when administered at 0.1ppm (100ppb) in a bath solution. However, the 0.05ppm (50ppb) dose was only 60% effective. WO1999/63824 described injectable compositions of milbemycin derivatives (including milbemycin oxime), avermectins, and other antiparasitic agents. Fish were administered milbemycin oxime at a dose of 0.5mg/kg. Milbemycin oxime was not efficacious since it was purported that only hexaflumuron (50mg/kg), lufenuron (50mg/kg), and moxidectin (0.2mg/kg) provided efficacy against sea lice. Surprisingly, Applicant discovered that milbemycin oxime is efficacious against sea lice at low doses on fish when administered to fish as a bath immersion.

Accordingly, a need still exists for new treatment options to control sea lice infestations on fish, particularly in farmed fish populations, that is safe and selective against the target parasite and is capable of treating sea lice populations showing resistance or reduced sensitivity to the current products. The present invention provides a new treatment option for controlling sea lice on fish.

### Summary

The present invention is defined in the appended claims. The present disclosure describes the use and methods of treatment for controlling sea lice infestations on fish by using milbemycin oxime, particularly as an immersion mode of administration.

In one aspect of the invention, is the use of milbemycin oxime for controlling parasitic infestations on fish comprising immersion of fish in water containing a therapeutically effective amount of milbemycin oxime. The effective amount of milbemycin oxime ranges from about 5ppb to about 500ppb as a concentration in water for treating fish. In yet another aspect of the invention, a therapeutically effective amount of milbemycin oxime ranges from about 5ppb to about 500ppb as a concentration in water for treating fish. In yet another aspect of the invention, a therapeutically effective amount of milbemycin oxime ranges from about 5ppb to about 200ppb as a concentration in water for treating fish. In yet another aspect of the invention, a therapeutically effective amount of milbemycin oxime ranges from about 5ppb to about 100ppb as a concentration in water for treating fish. In yet another aspect of the invention, a therapeutically effective amount of milbemycin oxime ranges from about 5ppb to about 90ppb as a concentration in water for treating fish. In yet another aspect of the invention, a therapeutically effective amount of milbemycin oxime ranges from about 5ppb to about 80ppb as a concentration in water for treating fish. In yet another aspect of the invention, a therapeutically effective amount of milbemycin oxime ranges from about 5ppb to about 60ppb as a concentration in water for treating fish. In yet another aspect of the invention, a therapeutically effective amount of milbemycin oxime ranges from about 5ppb to about 40ppb as a concentration in water for treating fish. In yet another aspect of the invention, a therapeutically effective amount of milbemycin oxime ranges from about 5ppb to about 25ppb as a concentration in water for treating fish. In yet another aspect of the invention, a therapeutically effective amount of milbemycin oxime ranges from about 10ppb to about 40ppb as a concentration in water for treating fish. In yet another aspect of the invention, a therapeutically effective amount of milbemycin oxime ranges from about 10ppb to about 25ppb as a concentration in water for treating fish. In yet another aspect of the invention, a therapeutically effective amount of milbemycin oxime ranges from about 5ppb, about 10ppb, about 15ppb, about 20ppb, about 25ppb, about 30ppb, about 35ppb, about 40ppb, about 45ppb, or about 50ppb in water for treating fish. In addition to the effective amounts listed above, it is also contemplated to have fractional amounts of milbemycin oxime, for example, 5.5ppb, 7.8ppb, 15.6ppb, 31.3ppb, 62.5ppb, and the like.

According to the invention, fish are farmed fish. Further according to the invention, farmed fish are salmonids. Preferred salmonids include, but are not limited to Atlantic salmon, rainbow trout, and sea trout.

Also according to the invention, the parasitic infestation is caused by a parasitic crustacean, particularly sea lice in the family, Caligidae. Preferred sea lice include, but are not limited to sea lice in the *Dissonus, Lepeophtheirus,* or *Caligus* genus. More preferred sea lice include the *Lepeophtheirus* or *Caligus* genus. In yet another aspect of the invention, the sea lice are copepodites, pre-adult sea lice, or adult sea lice.

In yet another aspect of the invention milbemycin oxime is used in a method of controlling a parasitic infestation on fish, the method comprising administering a therapeutically effective amount of milbemycin oxime to the fish by bath immersion. According to the invention the fish is a farmed fish. Further according to the invention, the farm fish is a salmonid. Preferred salmonids include, but are not limited to Atlantic salmon, rainbow trout, and sea trout.

According to the invention, the parasitic infestation is caused by a parasitic crustacean species that is sea lice, particularly sea lice in the family, *Caligidae.* Preferred sea lice are in the *Dissonus, Lepeophtheirus,* or *Caligus* genus. Even more preferred sea lice are *Lepeophtheirus salmonis oncorhynchi, Lepeophtheirus salmonis salmonis* or *Caligus elongatus.*

In yet another aspect of the invention, is a composition comprising milbemycin oxime for dilution for use as a bath immersion treatment for controlling a parasitic infestation on fish from sea lice. According to the invention, the composition is a stock solution of milbemycin oxime for dilution in a volume of water to be used as a bath immersion treatment for controlling a parasitic infestation on a fish from sea lice. Further according to the invention, the stock solution of milbemycin oxime comprises a solvent, and optionally, a solubilizer. In yet another aspect of the invention, the solvent is a non-aqueous polar solvent. In yet another aspect of the invention, the non-aqueous polar solvent is selected from the group consisting of methanol, ethanol, benzyl alcohol, isopropanol, acetone, methylene chloride, butyl diglycol, N-methyl-2-pyrrolidone, dimethylacetamide, dimethylformamide, dimethyl sulfoxide, polyoxyethylated ether, propylene glycol, ethylene glycol, and mixtures thereof. **In** yet another aspect of the invention, the non-aqueous polar solvent is selected from ethanol, benzyl alcohol, isopropanol, acetone, butyl diglycol, N-methyl-2-pyrrolidone, dimethylacetamide, dimethylformamide, dimethyl sulfoxide, and mixtures thereof. **In** yet another aspect of the invention, the non-aqueous polar solvent is selected from benzyl alcohol, butyl diglycol, N-methyl-2-pyrrolidone, dimethyl sulfoxide, and mixtures thereof. **In** one aspect of the invention, the non-polar aqueous solvent is dimethyl sulfoxide.

**In** yet another aspect of the invention, the composition further comprises a solubilizer. In yet another aspect of the invention, the solubilizer is selected from the group consisting of a polyoxyethylene castor oil derivative, polysorbate, caprylic/capric glyceride, poloxamer, polyoxyethylene alkyl ether, polyoxylglyceride, sorbitan fatty acid ester, polyoxyethylated 12-hydroxystearic acid, propylene glycol esters, polyglycerol esters, polyvinylpyrrolidone, cyclodextrin, polyethylene glycols, glyceryl stearates, caprylic glycerides, glyceryl monooleate, capric glycerides, alcohol ethoxylates, and mixtures thereof. In yet another aspect of the invention, the solubilizer is a polysorbate or a polyoxyl castor oil. In yet another aspect of the invention, the polysorbate is polysorbate 80. In yet another aspect of the invention, the polyoxyl castor oil is polyoxyl 35 castor oil. In yet another aspect of the invention, the milbemycin can be dissolved in a solubilizer alone.

### Discussion

### Definitions:

"About" when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater.

"Control" or "Controlling", as used herein, refers to reducing the number of sea lice, eliminating sea lice and/or preventing further sea lice infestation.

"Fish" as used herein, refers to food fish, breeding fish and aquarium, pond fish, and farmed fish of all ages occurring in freshwater, sea water (e.g., marine) and brackish water; more particularly, to marine fish, and more particularly to marine food fish. Non-limiting examples of food fish include: carp, eel, trout, whitefish, salmon, roach, rudd, chub, arctic char, sturgeon, plaice, halibut, turbot, flounder, striped bass, yellowtail, grouper, cod, sole, tuna, red sea bream, sea bass, grey mullet, pompano, gilthread seabream, tilapia, and catfish.

"Parasiticide", as used herein, refers to any substance that is capable of depleting the parasite population, for example by killing or preventing growth or reproduction of the parasites, or otherwise causing the loss or removal of parasites from the host fish. The substance may target a broad range of parasites, or may be specific for a small group of parasites, such as an individual type of parasite.

"Resistance", particularly "emamectin resistance", as used herein, refers to reduced potency of a compound as compared to naïve parasites, particularly sea lice.

"Sea lice" as used herein, unless otherwise indicated, refers to parasitic crustaceans (copepods) which feed through or upon the mucus and skin of its host, and are within the order *Siphonostomatoida,* family *Caligidae.*

"Therapeutically effective amount", as used herein, unless otherwise indicated, refers to an amount of milbemycin oxime that (i) treat the particular parasitic infestation, (ii) attenuates, ameliorates, or eliminates one or more symptoms of the particular parasitic infestation, or (iii) prevents or delays the onset of one or more symptoms of the particular parasitic infestation described herein.

"Treat", "Treating", "Treatment" and similar terms, are intended to refer to prophylactic or responsive treatment, such as the control, elimination, protection against, and/or prevention of the fish parasite (such as sea lice) infestation or condition in a fish population. The terms encompass reducing the mean number of parasites (such as sea lice) infesting each fish in a fish population; preventing an increase in the mean number of parasites infesting each fish in a fish population; preventing the onset of an infestation with parasites (such as sea lice), or of symptoms associated with a parasitic infestation, including reducing the severity of a disorder or condition or symptoms associated with the infestation. The terms also encompass preventing the recurrence of a fish parasite infestation or of symptoms associated therewith as well as references to "control" (such as, for example, kill, repel, expel, incapacitate, deter, eliminate, alleviate, minimize, and eradicate). Reference to treating or controlling a parasitic infestation "on" a fish is understood to constitute treatment of an external parasite, such as sea lice, which feeds "on" a fish and not necessarily exist inside the fish. However, it is also contemplated that the milbemycin oxime can treat parasites within and around the gills of a fish.

"Veterinary acceptable" as used herein, unless otherwise indicated, indicates that the substance or composition must be compatible chemically and/or toxicologically, with the other ingredients comprising a composition and/or the fish being treated therewith.

Sea lice are ectoparasites that belong to the sub-class of copepoda which affect fish, particularly farmed salmonids, negatively by feeding on the mucus, skin, and blood of the fish host. Sea lice can cause significant harm (i.e., serious fin damage, skin erosion, bleeding and open wounds) to host fish. Additionally, sea lice can cause chronic stress response in fish, which in turn can make them susceptible to other diseases. In addition, it appears that the sea lice have immunomodulatory effects on the host fish and can function as a vector in the transmission of other fish diseases. Damages due to parasitic infestations from sea lice result in considerable fish losses and increased expense. Infestation with sea lice is considered one of the most important disease problems in the farming of salmonids, especially Atlantic salmon (*Salmo salar*) and rainbow trout (*Oncorhynchus mykiss*). Infestation with sea lice can also occur in other fish species, for example, sea bass, tilapia, carp, and the like. In addition to the costs that are associated with treatment, lower classification ratings of slaughtered fish and reduced growth rate due to reduced feed intake contribute to the economic losses.

Sea lice are parasitic crustaceans (copepods) which feed through or upon the mucus and skin of its host, and are within the order *Siphonostomatoida,* family *Caligidae.* Two representatives from the family cause substantial losses in yield: *Lepeophtheirus* and *Caligus. Lepeophtheirus* has a brown, horseshoe-shaped carapace and *Caligus* is also brown, but smaller. Species within *Lepeophtheirus* include *Lepeophtheirus salmonis oncorhynchi, Lepeophtheirus salmonis salmonis* and within *Caligus* include *Caligus clemensi, Caligus curtus, Caligus dussumieri, Caligus elongatus, Caligus longicaudatus, Caligus rogercresseyi* and *Caligus stromii.* Sea lice also include the Copepods [hoppers] of the genera *Ergasilus; Bromolochus; Chondracaushus; Elythrophora; Dichelestinum; Lamproglenz; Hatschekia; Legosphilus; Symphodus; Ceudrolasus; Pseudocycmus; Lemaea; Lemaeocera; Pennella; Achthares; Basanistes; Salmincola; Brachiella; Epibrachiella; Pseudotracheliastes; and the families: Ergasilidae; Bromolochidae; Chondracanthidae; Calijidae; Dichelestiidae; Philichthyidae; Pseudocycnidae; Lernaeidae; Lernaepotidae; Sphyriidae;* and *Cecropidae.* Sea lice also includes parasitic crustaceans in the order *Isopoda,* for example, *Ceratothoa oestroides* and *Anilocra physodes.* Sea lice also includes substantially the free swimming larval stages of the respective pre-adults and adults.

In a preferred aspect of the invention, the fish are kept in sea water tanks, cages or nets. The cages and nets are moored in sea inlets such that a daily tidal flow of water passes through them to ensure a sufficient supply of oxygen and clean water. For tanks, there is a continual flow of sea water in and out of the tanks or at least scheduled flushing of fresh sea water to ensure sufficient water quality and oxygen to maintain fish health. In this artificial environment, the fish are fed and, if necessary, provided with medication until they mature sufficiently for marketing as edible fish or are selected for further breeding. Extremely intensive cage stocking is maintained in these fish farms. In this pure monoculture, the exceedingly high fish densities coupled with the other stress factors cause the caged fish to become in general markedly more susceptible to disease, epidemics and parasites than their free-living co-species. To maintain healthy populations, the farmed fish are treated regularly with bactericides (in certain countries) and/or antiparasitics, and are monitored.

Within the scope of this invention will be understood as comprising all representatives of the family Salmonidae, especially of the subfamily *salmonini* and, preferably, the following species: *Salmo salar* (Atlantic salmon); *Salmo trutta* (brown or sea trout); *Oncorhynchus mykiss* (rainbow trout); and the Pacific salmon (*Oncorhynchus*): *O. gorbuscha;* O. *keta;* O. *nekra; O. kisutch, O. tshawytscha* and *O*. *mason;* also comprised are artificially propagated species such as *Salvelinus* species and *Salmo clarkia.*

A particular parasiticide, milbemycin oxime, is represented by the following formula: 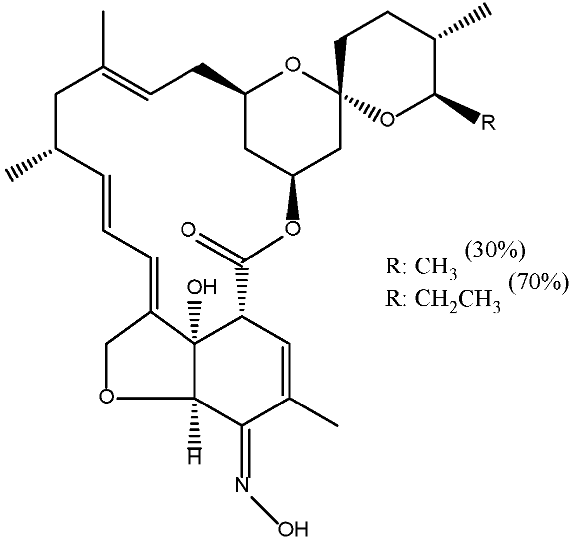 Milbemycin oxime is a semi-synthetic milbemycin used as a broad spectrum antiparasitic. It is generally used as an anthelmintic (worms) and miticide (mites). Milbemycins are products of fermentation by Streptomyces species. The milbemycins function by opening glutamate sensitive chloride channels in neurons and myocytes of invertebrates, leading to hyperpolarization of these cells and blocking signal transfer. It is commercially supplied as a crystalline solid. The solid can be dissolved in a polar solvent for example, ethanol, methanol, DMSO, DMF, NMP, acetone, and the like. Milbemycin oxime can also be dissolved in a polar solvent in combination with a solubilizer, for example Kolliphor (polyoxyl castor oil). Milbemycin oxime is also partially miscible in water. As stated, milbemycin oxime can be dissolved in a polar solvent with or without a solubilizer to prepare a solution prior to the addition to water for bath application or for treating the biotope. Milbemycin oxime can also be prepared or formulated in any suitable form, such as powder, granulate, emulsion, micro/nanoemulsion, emulsifiable concentrate, suspension, nanosuspension, or suspension concentrate. The user may use these formulations in diluted or undiluted form. The most suitable form will be known to the skilled person and may be dependent on the exact circumstances of the administration, for example, the degree of dilution, stability of the composition, volume of water, number of fish, and the like. Of interest, are milbemycin oxime formulations for administration to fish by bath treatment that may be in liquid form, and may be prepared in any suitable manner.

For the avoidance of doubt, as used herein, the term "polar solvent" does not encompass the use of water as a solvent, and for clarity, the polar solvent may therefore be referred to as a "non-aqueous polar solvent". A solvent is considered to be a polar solvent if the solvent molecules have a permanent separation of positive and negative charges, or the centres of positive and negative charges do not coincide. Polar solvents have high dielectric constants, for example, a solvent with a dielectric constant above about 15.0 is generally considered to be a polar solvent. Non-exclusive examples of polar solvents include: SD alcohol 3A (denatured), methanol, ethanol, benzyl alcohol, isopropanol, acetone, methylene chloride, butyl diglycol, N-methyl-2-pyrrolidone (NMP, Pharmasolve^{®}), dimethylacetamide (DMA), dimethylformamide (DMF), dimethyl sulfoxide (DMSO), polyoxyethylated ethers (for example, ethylene glycol monomethyl ether, diethylene glycol monomethyl ether, diethylene glycol monoethyl ether, dipropylene glycol monomethyl ether, dipropylene glycol monoethyl ether, and the like), propylene glycol, ethylene glycol, and the like, and mixtures thereof.

In the present context, the term "solubilizer" refers to any substance that increases the solubility of milbemycin oxime, for example when it is used in a polar solvent or a mixture of polar solvents. The solubilizer may be an amphiphilic molecule or a surfactant, and could be cationic, anionic or non-ionic, non-polymeric or polymeric. Solubilization is a process in which the solubility of a compound is increased by the formation of micellar structures after addition of a solubilizer. The term solubilisation is almost exclusively used for aqueous systems. In aqueous pharmaceutical formulations, for instance, solubilizers are often used to facilitate the dissolution of poorly soluble compounds. A solubilizer may be used in the manufacture of an aqueous milbemycin oxime formulation, for example for use in a bath treatment. Various solubilizers may be used in the aqueous solution formulation (i.e., stock solution). A suitable solubilizer can be selected from the list of solubilizers or emulsifiers given in the Handbook of pharmaceutical additives, M. Ash and I. Ash, Gower publishing limited, 1996, page 900-903 or 874-880, respectively. The term solubilizer also encompasses naturally derived and semisynthetic/synthetic monoglycerides, diglycerides, and triglycerides. Non-exclusive examples of suitable solubilizers include: glyceryl hexanoates such as Captex^{®} (e.g., 200, 300, 350, 355, 500, 800, and the like), glyceryl monolineleate, polyoxyethylene sorbitane fatty acid esters such as polyoxyethylene castor oil derivatives such as polyoxyl 5 castor oil, polyoxyl 9 castor oil, polyoxyl 15 castor oil, polyoxyl 35 castor oil (Cremophor^{®} EL; Kolliphor EL^{®}), polyoxyl 40 castor oil, polyoxyl 40 hydrogenated castor oil, polyoxyl 60 castor oil, polyoxyl 60 hydrogenated castor oil, polyoxyl 100 castor oil, polyoxyl 100 hydrogenated castor oil, polyoxyl 200 castor oil and polyoxyl 200 hydrogenated castor oil; polysorbates, for example, polysorbate 20 (Tween^{®}20), polysorbate 21, polysorbate 40, polysorbate 60, polysorbate 61, polysorbate 65, polysorbate 80 (Tween^{®}80), polysorbate 81, polysorbate 85 and polysorbate 120; caprylic/capric glycerides (e.g., Miglyols (e.g., 810, 812, 818, 829, 840, and the like); poloxamers (polyoxyethylene - polyoxypropylene copolymers) such as poloxamer 124, poloxamer 188, poloxamer 237, poloxamer 338 and poloxamer 407; polyoxyethylene alkyl ethers (e.g., Cetomacrogol 1000, polyoxyl 6 cetostearyl ether, polyoxyethylene(10) cetyl ether (Brij^{®}C10), polyoxyl 20 cetostearyl ether, polyoxyl 25 cetostearyl ether, polyoxyl 2 cetyl ether, polyoxyl 10 cetyl ether, polyoxyl 20 cetyl ether, polyoxyl 4 lauryl ether, polyoxyl 9 lauryl ether, polyoxyl 23 lauryl ether, polyoxyl 2 oleyl ether, polyoxyl 10 oleyl ether, polyoxyl 20 oleyl ether, polyoxyl 2 stearyl ether, polyoxyl 10 stearyl ether, polyoxyl 21 stearyl ether and polyoxyl 100 stearyl ether; polyoxylglycerides (e.g., caprylocaproyl polyoxylglycerides, lauroyl polyoxylglycerides, linoleoyl polyoxylglycerides, oleoyl polyoxylglycerides and stearoyl polyoxylglycerides); sorbitan fatty acid esters (e.g., sorbitan diisostearate, sorbitan dioleate, sorbitan monoisostearate, sorbitan monolaurate, sorbitan monooleate (Span^{®} 80), sorbitan monopalmitate, sorbitan monostearate, sorbitan sesquiisostearate, sorbitan sesquioleate, sorbitan sesquistearate, sorbitan triisostearate, sorbitan trioleate and sorbitan tristearate); macrogol 15 hydroxystearate, cetostearyl alcohol, cetyl alcohol, polyoxyethylated 12-hydroxystearic acid (Kolliphor^{®}HS15), propylene glycol esters, polyglycerol esters, hypromellose acetate succinate, polyvinylpyrrolidone, cyclodextrins, mono- and diesters of hydroxystearic acids and macrogols (e.g., macrogolglycerol hydroxystearate (Kolliphor^{®}RH40 )), polyethylene glycols, glyceryl stearates, glyceryl cocoate, caprylic glycerides, glyceryl monooleate, glyceryl ricinoleate, capric glycerides, alcohol ethoxylates, and the like. Preferred solubilizers include Tween^{®}80 and Kolliphor^{®}EL.

When the milbemycin oxime formulation comprises a solubilizer, the solubilizer is generally used in the amount of about 5-60% (w/w). Preferably, the formulation comprises 10-55% (w/w) solubilizer, more preferably 15-50% (w/w) solubilizer, such as, for example, about 15, 20, 25, 30, 35, 40, 45, 50 or 55% (w/w) solubilizer. The milbemycin oxime formulation may comprise a mixture of two or more different solubilizers.

In addition, or alternatively, the liquid milbemycin oxime formulation may comprise a preservative. Any suitable preservative or combination of preservatives may be used, including, for example, chlorobutanol, benzyl alcohol, a paraben (such as methylparaben or propylparaben), sorbic acid, phenoxyethanol, thiomersal, butylated hydroxyanisole (BHA), butylated hydroxytoluene (BHT), and the like. The preservative may be included in the formulation in an amount of from 0.001 % to 2.5 % (w/v), such as from 0.002 to 1.5% (w/v), or from 0.02 to 1 (w/v).

The milbemycin oxime stock solutions can be used to prepare immersion baths with different concentrations of milbemycin oxime to achieve the dosing concentration required. The stock solution can be diluted at least once before mixing into water or can be poured directly into the volume of water for the treatment of fish.

The liquid milbemycin oxime formulation may comprise one or more additional parasiticides within the group of acetylcholine esterase (AchE) inhibitors, GABA-gated chloride channel antagonists, GABA-gated chloride channel inhibitors, sodium channel modulators, nicotinic acetylcholine receptor (nAChR) agonists, nicotinic acetylcholine receptor (aAChR) allosteric activators, chloride channel activators, juvenile hormone mimics, modulators of chordontonal organs, inhibitors of mitochondrial ATP synthase, uncouplers of oxidative phosphorylation via disruption of the proton gradient, nicotinic acethylcholine receptor (nAChR) channel blockers, inhibitors of chitin biosynthesis, type 0 and type 1, moulting disruptor, ecdysone receptor agonists, octopamine receptor agonists, mitochondrial complex III electron transport inhibitors, mitochondrial complex I electron transport inhibitors, voltage-dependent sodium channel blockers, inhibitors of acetylCoA carboxylase, mitochondrial complex II electron transport inhibitors, ryanodine receptor modulators, tubulin binders, acetylcholineesterase (AChE) mimetics, un-couplers of the oxidative phosphorylation. Non-limiting examples of the one or more additional parasiticides include: diafenthiuron, deltamethrin, indoxacarb; emamectin, ivermectin, moxidectin, nitenpyram, cyromazin, pymetrozine bistrifluron, chlorfluazuron, flucycloxuron, flufenoxuron, hexaflumuron, novaluron, noviflumuron, buprofezin, diflubenzuron, fluazuron, lufenuron, and teflubenzuron. A preferred additional antiparasitic agent includes: diflubenzuron, lufenuron, and hexaflumuron. The one or more additional parasiticides that may be present in the combination formulation together with milbemycin oxime may be present in the free form, or in any active form, such as in the form of any veterinary acceptable salt.

A suitable combination treatment comprising milbemycin oxime and an additional parasiticide, may be performed, for example, by treating the fish, in particular salmon, initially with the milbemycin oxime formulation, and thereafter, for example 1 week to 3 months or more, preferably 3 to 5 months or more, more preferably 6 months or more and in particular 8 to 12 months after the end of the milbemycin oxime treatment.

According to an aspect this combination treatment, the first treatment is the bath treatment with milbemycin oxime followed with an in-feed treatment with hexaflumuron, lufenuron, or emamectin that may provide lasting protection against sea lice. According to another aspect this combination treatment, the first treatment is an in-feed treatment with hexaflumuron, lufenuron, or emamectin that may provide lasting protection against sea lice followed by the bath treatment with milbemycin oxime.

Milbemycin oxime can be administered to fish by bath treatment, for example by placing the fish into a "medicinal bath" and keeping them there for a period of time (minutes to several hours), for example, when being transferred from one net pen or breeding basin to another. It is also possible to treat the biotope of the fish temporarily or continuously, such as, for example, the net cages, entire ponds, aquaria, tanks or basins in which the fish are kept. For use as a bath treatment, milbemycin oxime or a formulation comprising milbemycin oxime can be dissolved or suspended in the water containing the fish and/or parasite.

Milbemycin oxime is be used at a therapeutically effective amount or concentration of about 5ppb to about 500ppb; or from about 5ppb to about 250ppb; or from about 5ppb to about 200ppb; or from about 5ppb to about 100ppb; or about 5ppb to about 90ppb; or about 5ppb to about 80ppb; or about 5ppb to about 60ppb; or about 5ppb to about 50ppb; or about 5ppb to about 40ppb; or about 5ppb to about 25ppb; or about from about 10ppb to about 100ppb; or about 10ppb to about 80ppb; or about 10ppb to about 60ppb; or about 10ppb to about 50ppb; or about 10ppb to about 40ppb; or about 10ppb to about 25ppb; or about 10ppb; or about 15ppb; or about 20ppb; or about 25ppb, or about 30ppb, or about 35ppb, or about 40ppb, or about 45ppb, or about 50ppb in water. All effect concentrations are ppb as measured in a volume of water (fresh, salt, brackish) for treating fish against a copepod crustacean species, particularly, sea lice. It is also contemplated to have fractional amounts of milbemycin oxime, for example, 5.5ppb, 7.8ppb, 15.6ppb, 31.3ppb, 62.5ppb, and the like; or set dose amounts of 5ppb, 7ppb, 12ppb, 17ppb, 22ppb, 28ppb, 33ppb, 37ppb, 42ppb, 48ppb, 53ppb, 55ppb, 58ppb, 60ppb, 62ppb, 65ppb, 68ppb, 70ppb, 72ppb, 75ppb, 78ppb, 80ppb, 83ppb, 85ppb, 87ppb, 90ppb, 93ppb, 95ppb, 97ppb, 100ppb, and the like; based on total water volume. These values represent the concentration of milbemycin oxime in the water containing the fish (i.e. the actual levels needed to control the parasite population). The concentrations are achieved by adding a volume of the concentrated stock solution of milbemycin oxime, for example 50mg/mL or 100mg/mL, to the enclosure containing the fish. The skilled person can determine how much milbemycin oxime stock solution should be added from knowledge of the volume of the enclosure containing the fish and the concentration of the stock solution.

The concentration of milbemycin oxime during application to the fish depends on the manner and duration of treatment and also on the age and condition of the fish being treated. A typical immersion time ranges from about 15 minutes to about 4 hours, preferably from about 15 minutes to 2 hours, and more preferably from about 30 minutes to about 1 hour.

This method of administration is very simple and labor efficient, and has the added advantage that the milbemycin oxime may be able to act directly on parasites that are predominantly external to the fish, such as sea lice.

The invention also provides for a veterinary pack or kit comprising one or more containers filled with the formulation of the invention. Additionally, provided in the kit is notice in the form prescribed by a governmental agency regulating the manufacture, use or sale of the pharmaceuticals, and an instruction for use of the pharmaceutical, wherein said pharmaceutical is milbemycin oxime.

### Experimental

An earlier milbemycin derivative, VS55759 (CAS133824-53-0) with the following structure: 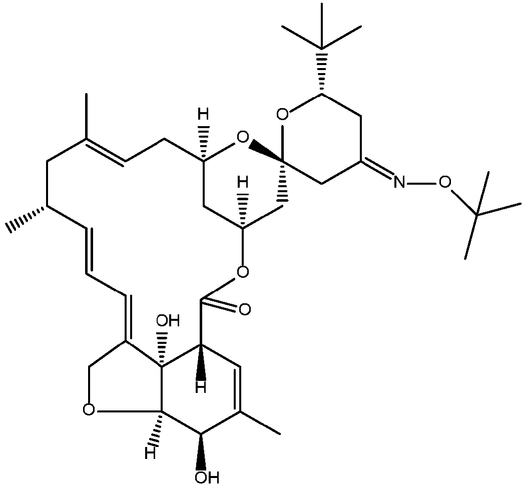 was used to treat fish against sea lice by immersion. According to WO1992/08352, the 100ppb dose was shown to be 100% effective against sea lice. However, the 50ppb dose was only found to be about 60% effective.

In a number of recent in-vitro studies, sensitive or reduced sensitivity salmon lice were placed in containers with sea water. Milbemycin oxime was formulated in a solution of acetone and Kolliphor^{®}EL (1:1) or NMP at 1mg/mL. Aliquots of the stock solution were pipetted into the sea water at concentrations ranging from 2ppb to 50ppb. Control solutions did not receive milbemycin oxime. At least 5 sea lice (in duplicate) were added to each container and were exposed to milbemycin oxime for 1 hour. In one study, copepodids from salmon lice with reduced sensitivity exposed to 50ppb, 25ppb, and 12.5ppb were moribund at 96 hours post-exposure. Control copepodids were all alive and normal. In a second in-vitro study, preadult salmon lice with reduced sensitivity were exposed to 5ppb milbemycin oxime and all were moribund at 48 hours post-exposure; while adult reduced sensitivity lice exposed to 10ppb and 25ppb were all dead or moribund over the same time period. Adult sea lice exposed to 2ppb had an 82% moribund rate at 48 hours post-exposure.

Considering VS55759 provided only 60% morbidity of sea lice at 50ppb, milbemycin oxime at a dose of 5ppb was unpredictably at least 10x more effective at killing sea lice than the milbemycin derivative described more than 26 years ago.

In a separate in-vivo study, Atlantic salmon infested with adult stages of L. *salmonis* were housed in a 500L tank with sea water at 12°C before drug exposure. Fish were randomly extracted from the holding tank and distributed to the treatment containers (n=14/container). Treated groups received a bath exposure with milbemycin oxime for one hour in 30L tanks. Fish were then transferred to observation tanks. Fish were dosed with 500ppb, 250ppb, 125ppb, and 62.5ppb milbemycin oxime. Stock solution of milbemycin oxime was 100mg/mL. On Day 3, fish were terminated and the number of female salmon lice were counted on each of the fish. Milbemycin oxime was 100% efficacious against the sea lice at all dose levels.

In a second in-vivo study, the efficacy of milbemycin oxime was assessed in a negative controlled and randomized study using Atlantic salmon smolts infested with salmon lice copepodids. Smolts had an average weight of about 89 grams and were all infested in a single 500L tank with a fish density of about 24 kg/m³ held at about 12°C. When the copepodids reached pre-adult stage (about 3-weeks (~ 252 degree days of development)), the fish (n=20/treatment group) received a bath exposure with milbemycin oxime for one hour in 60L tanks on Day 1. Fish were dosed at 62.5ppb, 31.3ppb, 15.6ppb, and 7.8ppb milbemycin oxime. Stock solution of milbemycin oxime was 100mg/mL. Fish were transferred to observation tanks. On Day 3, fish were terminated and sea lice (adults and pre-adults) were counted. Milbemycin oxime was shown to be 100% efficacious against adult sea lice at 62.5ppb, 31.3ppb, and 7.8ppb. Efficacy at 15.6ppb was 66.6%. The remaining living sea lice in the 15.6ppb group were found to be loosely attached to the caudal fin and easily removed.

## Claims

1. Milbemycin oxime for use in controlling parasitic infestations caused by sea lice in the *Caligidae* family on farmed salmonid fish, said use comprising immersion of fish in water containing a therapeutically effective amount of milbemycin oxime, and wherein the effective amount of milbemycin oxime in water is about 5ppb to about 500ppb.

2. The milbemycin oxime for use according to Claim 1, wherein the effective amount of milbemycin oxime in water ranges from about 5ppb to about 100ppb.

3. The milbemycin oxime for use according to Claim 2, wherein the effective amount of milbemycin oxime in water ranges from about 10 to about 40ppb.

4. The milbemycin for use according to Claims 1 to 3, wherein the sea lice are copepodites, pre-adult sea lice, or adult sea lice in the *Lepeophtheirus* or *Caligus* genus.

5. Milbemycin oxime for use in a bath immersion treatment for the control of a parasitic infestation on farmed salmonid fish, wherein the amount of milbemycin oxime in the water is about 5ppb to about 500ppb, and wherein the parasitic infestation is a parasitic crustacean species that is sea lice in the *Lepeophtheirus* or *Caligus* genus of the *Caligidae* family.

6. Milbemycin oxime for use of any one of claims 1 to 5, wherein a composition that is a stock solution comprising the milbemycin oxime is added to an enclosure containing the fish, wherein the composition comprises milbemycin oxime, a solvent and optionally a solubilizer.

7. Milbemycin oxime for use of Claim 6, wherein the solvent is a non-aqueous polar solvent selected from the group consisting of methanol, ethanol, benzyl alcohol, isopropanol, acetone, methylene chloride, butyl diglycol, N-methyl-2-pyrrolidone, dimethylacetamide, dimethylformamide, dimethyl sulfoxide, polyoxyethylated ether, propylene glycol, ethylene glycol, and mixtures thereof; and wherein the composition further comprises a solubilizer.

8. Milbemycin oxime for use of Claim 7, wherein the solubilizer is selected form the group consisting of a polyoxyethylene castor oil derivative, polysorbate, caprylic/capric glyceride, poloxamer, polyoxyethylene alkyl ether, polyoxylglyceride, sorbitan fatty acid ester, polyoxyethylated 12-hydroxystearic acid, propylene glycol esters, polyglycerol esters, polyvinylpyrrolidone, cyclodextrin, polyethylene glycols, glyceryl stearates, caprylic glycerides, glyceryl monooleate, capric glycerides, alcohol ethoxylates, and mixtures thereof.

9. Milbemycin oxime for use of Claim 8, wherein the non-aqueous polar solvent is selected from ethanol, benzyl alcohol, isopropanol, acetone, butyl diglycol, N-methyl-2-pyrrolidone, dimethylacetamide, dimethylformamide, dimethyl sulfoxide, and mixtures thereof; and the solubilizer is a polysorbate or a polyoxyl castor oil.

## Patentansprüche

1. Milbemycinoxim zur Verwendung bei der Bekämpfung von Parasitenbefall, der durch Seeläuse in der Familie der Caligidae auf Zuchtsalmonidenfischen verursacht wird, wobei die Verwendung das Eintauchen von Fischen in Wasser umfasst, das eine therapeutisch wirksame Menge an Milbemycinoxim enthält, und wobei die wirksame Menge an Milbemycinoxim in Wasser etwa 5 ppb bis etwa 500 ppb beträgt.

2. Milbemycinoxim zur Verwendung nach Anspruch 1, wobei die wirksame Menge an Milbemycinoxim in Wasser im Bereich von etwa 5 ppb bis etwa 100 ppb liegt.

3. Milbemycinoxim zur Verwendung nach Anspruch 2, wobei die wirksame Menge an Milbemycinoxim in Wasser im Bereich von etwa 10 bis etwa 40 ppb liegt.

4. Milbemycinoxim zur Verwendung nach den Ansprüchen 1 bis 3, wobei die Seeläuse Copepodite, voradulte Seeläuse oder adulte Seeläuse in der Gattung *Lepeophtheirus* oder *Caligus* sind.

5. Milbemycinoxim zur Verwendung bei einer Badetauchbehandlung zur Bekämpfung eines Parasitenbefalls auf Zuchtsalmonidenfischen, wobei die Menge an Milbemycinoxim in dem Wasser etwa 5 ppb bis etwa 500 ppb beträgt und wobei der Parasitenbefall eine Parasitenkrebstierart ist, die Seeläuse in der Gattung *Lepeophtheirus* oder *Caligus* der Familie der Caligidae ist.

6. Milbemycinoxim zur Verwendung nach einem der Ansprüche 1 bis 5, wobei eine Zusammensetzung, die eine Stammlösung ist, die das Milbemycinoxim umfasst, zu einer Umhüllung, die die Fische enthält, gegeben wird, wobei die Zusammensetzung Milbemycinoxim, ein Lösungsmittel und gegebenenfalls einen Lösungsvermittler umfasst.

7. Milbemycinoxim zur Verwendung nach Anspruch 6, wobei das Lösungsmittel ein nichtwässriges polares Lösungsmittel ist, das aus der Gruppe ausgewählt ist, die aus Methanol, Ethanol, Benzylalkohol, Isopropanol, Aceton, Methylenchlorid, Butyldiglykol, N-Methyl-2-pyrrolidon, Dimethylacetamid, Dimethylformamid, Dimethylsulfoxid, polyoxyethyliertem Ether, Propylenglykol, Ethylenglykol und Mischungen davon besteht; und wobei die Zusammensetzung ferner einen Lösungsvermittler umfasst.

8. Milbemycinoxim zur Verwendung nach Anspruch 7, wobei der Lösungsvermittler aus der Gruppe ausgewählt ist, die aus einem Polyoxyethylen-Rizinusöl-Derivat, Polysorbat, Capryl- /Capringlycerid, Poloxamer, Polyoxyethylenalkylether, Polyoxylglycerid, Sorbitanfettsäureester, polyoxyethylierter 12-Hydroxystearinsäure, Propylenglykolestern, Polyglycerinestern, Polyvinylpyrrolidon, Cyclodextrin, Polyethylenglykolen, Glycerylstearaten, Caprylglyceriden, Glycerylmonooleat, Capringlyceriden, Alkoholethoxylaten und Mischungen davon besteht.

9. Milbemycinoxim zur Verwendung nach Anspruch 8, wobei das nichtwässrige polare Lösungsmittel aus Ethanol, Benzylalkohol, Isopropanol, Aceton, Butyldiglykol, N-Methyl-2-pyrrolidon, Dimethylacetamid, Dimethylformamid, Dimethylsulfoxid und Mischungen davon ausgewählt ist; und der Lösungsvermittler ein Polysorbat oder ein Polyoxyl-Rizinusöl ist.

## Revendications

1. Oxime de milbémycine pour une utilisation dans la lutte contre les infestations parasitaires causées par les poux de mer de la famille *Caligidae* sur des poissons salmonidés d'élevage, ladite utilisation comprenant l'immersion des poissons dans de l'eau contenant une quantité thérapeutiquement efficace d'oxime de milbémycine, et dans laquelle la quantité efficace d'oxime de milbémycine dans l'eau est d'environ 5 ppb à environ 500 ppb.

2. Oxime de milbémycine pour une utilisation selon la revendication 1, dans laquelle la quantité efficace d'oxime de milbémycine dans l'eau va d'environ 5 ppb à environ 100 ppb.

3. Oxime de milbémycine pour une utilisation selon la revendication 2, dans laquelle la quantité efficace d'oxime de milbémycine dans l'eau va d'environ 10 à environ 40 ppb.

4. Milbémycine pour une utilisation selon les revendications 1 à 3, dans laquelle les poux de mer sont des copépodites, des poux de mer pré-adultes ou des poux de mer adultes du genre *Lepeophtheirus* ou *Caligus.*

5. Oxime de milbémycine pour une utilisation dans un traitement par immersion dans un bain pour lutter contre une infestation parasitaire sur des poissons salmonidés d'élevage, dans laquelle la quantité d'oxime de milbémycine dans l'eau est d'environ 5 ppb à environ 500 ppb, et dans laquelle l'infestation parasitaire est une espèce de crustacés parasites, à savoir des poux de mer du genre *Lepeophtheirus* ou *Caligus* de la famille *Caligidae.*

6. Oxime de milbémycine pour une utilisation selon l'une des revendications 1 à 5, dans laquelle une composition sous forme de solution mère comprenant l'oxime de milbémycine est ajoutée à un bassin contenant les poissons, dans laquelle la composition comprend de l'oxime de milbémycine, un solvant et optionnellement un solubilisant.

7. Oxime de milbémycine pour une utilisation selon la revendication 6, dans laquelle le solvant est un solvant polaire non aqueux sélectionné dans le groupe consistant en le méthanol, l'éthanol, l'alcool benzylique, l'isopropanol, l'acétone, le chlorure de méthylène, le butyldiglycol, la N-méthyl-2-pyrrolidone, le diméthylacétamide, le diméthylformamide, le diméthylsulfoxyde, un éther polyoxyéthylé, le propylène-glycol, l'éthylène-glycol, et des mélanges de ceux-ci ; et dans laquelle la composition comprend en outre un solubilisant.

8. Oxime de milbémycine pour une utilisation selon la revendication 7, dans laquelle le solubilisant est sélectionné dans le groupe consistant en un dérivé d'huile de ricin polyoxyéthyléné, un polysorbate, un glycéride caprylique/caprique, un poloxamère, un alkyléther polyoxyéthyléné, un polyoxylglycéride, un ester de sorbitane d'acide gras, un acide 12-hydroxystéarique polyoxyéthyléné, les esters de propylène-glycol, les esters de polyglycérol, une polyvinylpyrrolidone, une cyclodextrine, les polyéthylène-glycols, les stéarates de glycéryle, les glycérides capryliques, le monooléate de glycéryle, les glycérides capriques, les éthoxylates d'alcool, et des mélanges de ceux-ci.

9. Oxime de milbémycine pour une utilisation selon la revendication 8, dans laquelle le solvant polaire non aqueux est sélectionné parmi l'éthanol, l'alcool benzylique, l'isopropanol, l'acétone, le butyldiglycol, la N-méthyl-2-pyrrolidone, le diméthylacétamide, le diméthylformamide, le diméthylsulfoxyde et des mélanges de ceux-ci ; et le solubilisant est un polysorbate ou une huile de ricin polyoxylée.
